# EUROPEAN PATENT APPLICATION

(11) **EP 1 598 002 A1**
(43) Date of publication of application: **23.11.2005**
(21) Application number: 04425364.9
(22) Date of filing: 20.05.2004
(51) Int. Cl.: A61B 1/32

(54) **Medical device for vaginal and rectal examinations or operations**

(71) Applicant: Sapi Med S.p.A., 15100 Alessandria (IT)
(72) Inventor: Oddenino, Gian Paolo, 15100 Alessandria (IT)
(74) Representative: Long, Giorgio

(57) **Abstract**

A medical device for vaginal and rectal examinations or operations comprises dilating means (2) including a first (21) and second (22) hollow shells and an insertion means (3) receivable within the dilating means including an end portion (31) having a rounded shape to facilitate the insertion of the device.

## Description

The present invention relates to a medical device to be used for examinations /operations on body organs such as proctological or gynaecological operations. Particularly, the invention relates to a disposable medical device allowing both proctological and gynaecological examinations on a patient.

In the proctological medical practice, devices known as anoscopes or proctoscopes are known, which are suitable to allow the anus and the rectum to be examined by an operator. These devices generally consist of a first member comprising a hollow cylindrical portion ending with a first end provided with a handle to be manipulated and a second end, i.e. that for the insertion, which is moulded oblique and a second member consisting of a cylindrical portion suitable to be inserted and reversibly locked in said first member. The second element also displays an inserting end having a rounded tip which allows to facilitate the insertion of the device in the patient's body.

In the gynaecological medical practice, a device denominated speculum and essentially consisting of a portion formed by two symmetrical shells of a substantially cylindrical or conical shape, which end such as to form a "duck bill" and a portion provided with a handle for an operator to manipulate the device. These shells can be widened such as to act as a dilator.

For anatomical reasons, said devices are substantially different and hence they need to be used specifically and separately. In fact, the speculum with the flat and wide "duck bill" ends cannot be used as an anoscope because of the anal sphincter muscles. On the contrary, the anoscope can be uncomfortable for the patient while being inserted in the vagina due to the truncated and pervious cylindrical end. Furthermore, the anoscope cannot be used as an adjustable dilator.

The technical problem at the heart of the present invention is to provide a medical device to be used in a patient for example both for gynaecological and proctological purposes, with no need to employ different devices.

This problem is resolved by a medical device such as stated in the annexed independent claim.

Further characteristics and the advantages of the present invention will appear from the description below of an embodiment, which is given by way of a non-limiting example, wherein:
- Figure 1 is an exploded view of the medical device of the invention;
- Figure 2 is a perspective view of the device from Figure 1 when assembled;
- Figure 3 is a perspective view of the medical device from Figure 2 in a possible operative condition;
- Figure 4 is a partial perspective view of a detail from Figure 3;
- Figure 5 is a front view in partial section of a detail from Figure 3.

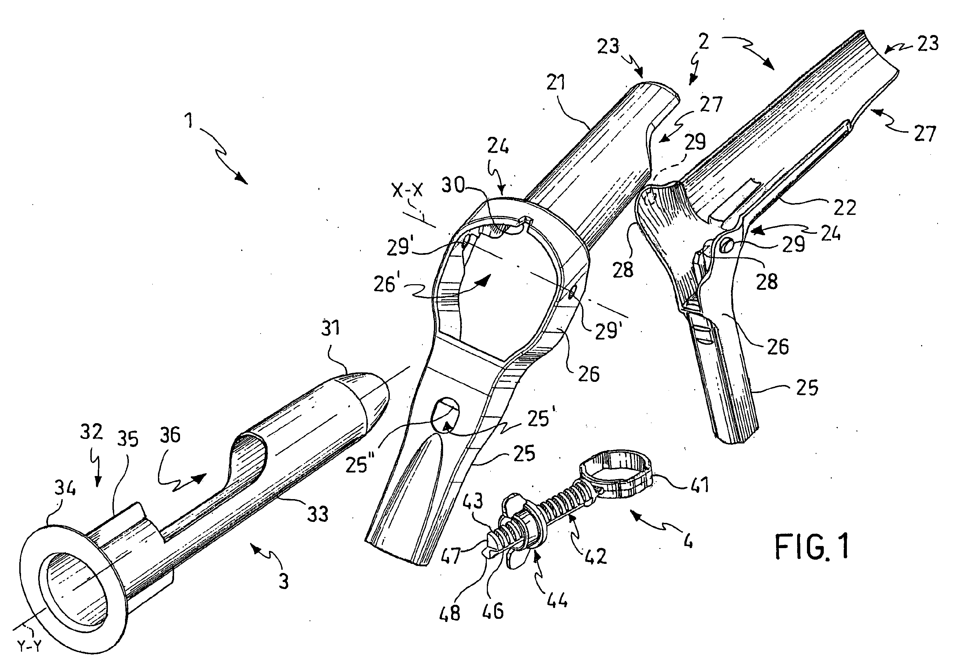

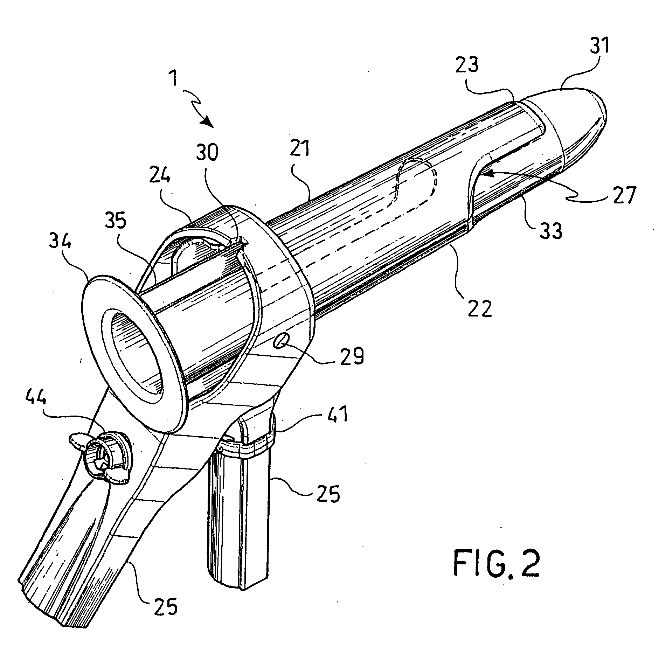

In Figures 1 and 2, with the numeral 1 is generally designated a medical device for gynaecological/proctological examinations according to the present invention.

The subject medical device 1 can be generally used for examinations/operations of organs and comprises dilating means 2 suitable to be inserted in an organ and maintain it wide open and inserting means 3 suitable to facilitate the insertion of said device in said organ.

The dilating means 2 comprise a first shell 21 and a second shell 22, both of them hollow, which are connected such as to move relative to one another. Preferably, the first 21 and second shells 22 have a semi-cylindrical shape, each one with a first free end 23 and a second end 24 integrally connected to a handle 25 by means of a joint portion 26 such as to form a sort of L. Particularly, the first shell 21 and the corresponding handle 25 form an inner angle greater than 90°, whereas the second shell 22 and the corresponding handle 25 form an inner angle smaller than or equal to 90°. Furthermore, said first 21 and second shells 22 have a smooth outer surface and, when closed, they define a hollow cylinder open at both first 23 and second 24 ends. Advantageously, the first 21 and second shells 22 are provided, at said first end 23, with a notch 27 the function of which will be described below with reference to the use of device 1.

As stated above, the first 21 and second shells 22 are mutually connected, preferably by means of a pin-slot engagement, such as to allow the shells to be open and closed. Particularly, the second shells 22 is provided with two respective flexible wings 28 protruding from the joining portion 26 and carry two projecting pins 29. These pins 29 couple with matching slots 29' formed on the joining portion 26 of the first shell 21. Thus, both first 21 and second shells 22 can be opened or closed by acting on the respective handles 25 thanks to a first degree lever mechanism provided with a fulcrum along the X-X axis by means of this pin-slot engagement, power applied by an operator's hand on the handle 25 and the resistance provided by the inner wall of the body organ.

The joining portion 26 of the first shell 21 further presents a through hole 26' communicating with the cavity defined by both first 21 and second shells 22 when they are closed. As will be discussed below, said through hole allows for the inserting means 3 to be inserted.

Preferably, the edge of said through hole 26' is provided with a groove 30 suitable to house a corresponding rib of the inserting means 3, as will be described in detail below.

In addition, the handle 25 of first shell 21 can also be provided by a through hole 25' suitable to house a pin of the locking means, as will be described below. Particularly, said through hole 25' comprises a radially extending tooth 25'' which is such to be engaged with the locking means in a manner that will be described below.

Advantageously, the medical device 1 of the invention also comprises inserting means 3. The inserting means are represented by a core 3, commonly defined as obturator or introducer, having a substantially cylindrical shape extending along a longitudinal axis Y-Y and is such to be reversibly inserted in said dilating means 2. Particularly, the core 3 comprises a first end portion 31 having a rounded shape to facilitate the insertion of said dilating means in an organ, a second end portion 32 and a connecting portion 33 between said first and second end portions.

Preferably, the connecting portion 33 exhibits a long and extended groove 36 which runs substantially through the whole length thereof and more than half the circumpherence thereof.

The second end portion 32 is preferably provided with an end flange 34 being the end-of-stroke stop when said core is inserted in said first 21 and second shells 22, when closed. Furthermore, the outer surface of the second end portion 32 is provided with a longitudinal rib 35 suitable to be engaged in the corresponding groove 30 formed on the joining portion 26 of the first shell 21, such as discussed above. Particularly, said rib acts as a centring means which allows the inserting means to be properly positioned inside the shells, i.e. they allow to position the core 3 in the first 21 and second 22 shells such that the groove 36 is covered by the wall of the shells themselves when they are in the closed position, i.e. in the position of insertion in the organ. In other words, the groove 36 faces the opposite direction from groove 27 which is formed near the end 23 of said shells.

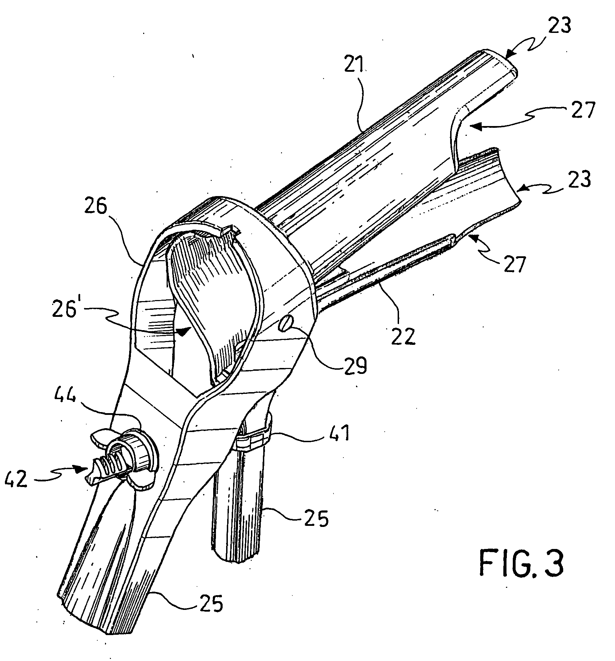

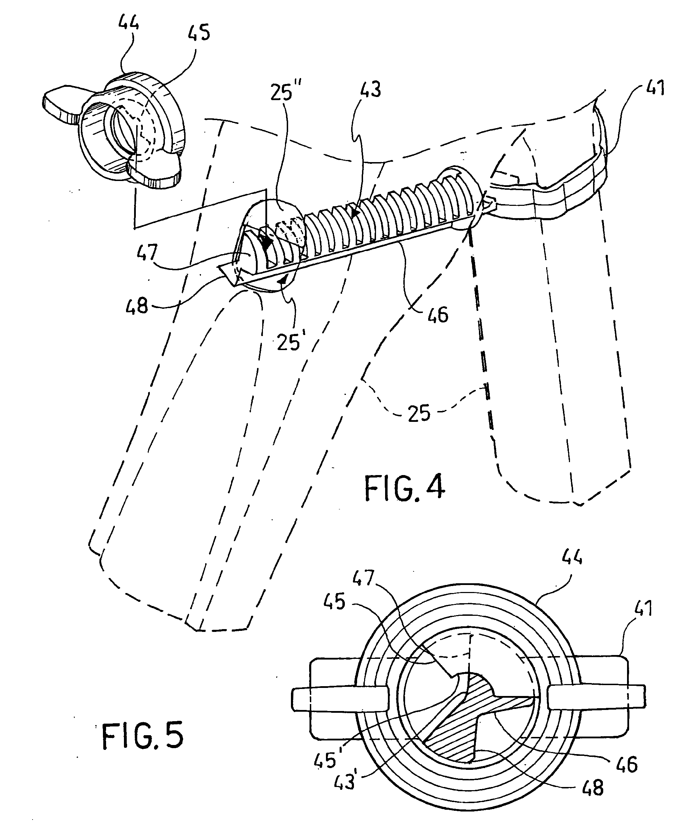

The device in accordance with the present invention can also comprise locking means 4, represented in detail in Figures 3 and 4, suitable to lock the first 21 and second shells 22 in an opening position as desired during an examination or operation. The advantage of said locking means is that it allows the operator to carry out examinations or operations such as biopsy and curettage with both hands free. To this purpose, the locking means can consist of a ring 41 integrally connected with a rigid pin 42 and a nut 44 of the wing-type.

Particularly, the ring 41 can be fit on the handle 25 of second shell 22 and releasably locked thereto for example by an annular seat (not shown) formed on the handle outer surface or simply by forced fitting on the handle.

The pin 42 comprises a longitudinal axial base 46 from which the slice-of-cake-shaped teeth 47 protrude and define a series of grooves 43. The bottom of each of said grooves 43 further has a relief 43' having a slice-of-cake sectional shape, such as shown in Figure 5. In a substantially diametrically opposite position to said teeth, said base 46 is run though the entire length thereof by a shoulder 48 suitable to act as the stop means for said nut 44.

The nut 44 is provided with a radially-extending relief 45 and is suitable to be engaged with said pin, such as will be described below. Particularly, the relief 45 comprises a circular notch 45' suitable to be engaged with the relief 43' of the grooves 43, such as will be described below.

The assembly of the medical device 1 first provides for the ring 41 to be fitted on the handle 25 of the second shell 22 such that the pin 42 faces the opposite direction from the shell itself. Next, the first shell 21 is mounted to the second shell 22 by bending the wings 28 inwardly to the shell 22 such as to allow the connecting portion 26 to overlap and provide a pin/slot snap engagement with the connecting portion of the first shell 21. At the same time, the pin 42 of the locking means 4 is caused to pass through the through hole 25' of the handle 25 of first shells 21. Now, the nut 44 is fitted on the pin 42 such that the relief 45 is between the tooth series 47 and the shoulder 48. By turning the nut 44 clockwise, the relief 45 thereof is inserted in a groove of the series of grooves 43 of pin 42 such that the notch thereof 45' engages the relief 43' of said groove. The nut 44 is thus locked on the pin to avoid that it may accidentally slip off. Finally, the obturator 3 is inserted between both first 21 and second 23 shells through the through hole 26' of connecting portion 26 of first shell 21 until the rib 35 thereof is engaged with groove 30 of said connecting portion 26 of the first shell 21. The medical device 1 is now ready to be packed and sold (Figure 2). Preferably, the device can be sterilized either before or after being packed.

The medical device 1 in accordance with the invention can be made with commonly used plastic materials for medical use such as polypropylene, polystyrene, HD polyethylene (high density) either disposable or multi-use. In the last case, the materials should be obviously sterilizable for example in ethylene oxide or by beta or gamma ray radiations and cleanable with proper detergents. Alternatively, the device can be manufactured with materials such as stainless steel or alloys for surgical instruments such that it can be cleaned and sterilized for repeated use.

The operation of the medical device in accordance with the present invention will be now described.

Particularly, the medical device 1 can be first used for a gynaecological examination and then for a proctological examination on the same patient.

With reference to the gynaecological examination, the medical device 1 can be generally sold already assembled such as illustrated above, i.e. with closed shells, with the obturator 3 being interposed therebetween and with the locking means 4 being already mounted thereon.

The medical device can be then inserted by the operator in the vagina from the first end 23 of the first 21 and second 22 shells from which the rounded end 31 of the core 3 protrudes. It is due to the shape of said rounded end that the insertion is much more comfortable for the operator and less uncomfortable for the patient than the conventional vaginal speculum.

Next, the obturator 3 is removed from the shells still being in the closed position to allow for examination or gynaecological operation at the vaginal level. Furthermore, the shells can be opened by acting on the respective handles to allow for a better operating condition. The opening position can also be adjusted and secured by acting on the locking means to be adjusted according to the needs or body characteristics of the patient. Particularly, after the shells have been opened to the desired position, the pin 42 is engaged with the handle 25 of first shell 21 by force fitting the tooth 25'' of said handle in a groove 43 of said pin. Therefore, the nut 44 is turned counter-clockwise to release its inner relief 45 from engagement with one of the grooves 43 of pin 42 and is caused to slide along said pin until being in abutment against the handle 25. Now, the nut is turned clockwise to cause said relief 45 to engage in one of grooves 43. Thus, the tooth 25'' of the handle 25 of the first shell 21 is locked in a groove 43 of the pin and, accordingly, the rigid pin 42 maintains a fixed position between the handles 25 of the first 21 and second 22 shells.

Once the operation or examination has been completed, the nut needs only to be unlocked 44 by turning it in the direction where the relief 45 thereof disengages from the grooves, cause the nut to slide away from the ring 41 to allow also the handles 25 to move away and the shells to close as a consequence.

As stated above, advantageously, the medical device 1 in accordance with the present invention can be used also for a proctological examination or operation, i.e. it can be used as an anoscope.

In fact, due to the specific shape of the first 21 and second 22 shells such as described above, the peculiar end 23 as well as the possibility of adjusting their mutual distance, the medical device 1 can be employed for the above purpose with no need to change device or parts thereof.

The use of the medical device 1 will be substantially identical to that already described above with reference to a gynaecological operation.

Furthermore, advantageously, the groove 27 of each shells allows to carry out biopsy, elastic ligatures and haemorrhoid sclerosant therapies, haematoma incision, suture with haemostatic stitches, photocoagulation using dedicated devices.

From what has been discussed above, it should be understood that the medical device in accordance with the present invention advantageously allows to use only one tool with a double function, i.e. gynaecological and proctologic, and hence to resolve the technical problem discussed in the introductory part.

Obviously, to the medical device according to the present invention, those skilled in the art, aiming at satisfying contingent and specific needs, will be able to carry out further modifications and variants, all of which are contemplated within the scope of protection of the invention, such as defined by the annexed claims.

For example, the shape of the shells can be of a truncated cone and the respective grooves 27 near the free end 23 can be more or less extended all along the wall of the shells and along the circumpherence.

The pin-slot engagement between the shells can also be replaced by different couplings that however allow to move both shells away such as to act as a dilator.

The substantially L-shaped shells with the corresponding handle can also be modified as desired or according to particular needs.

The locking means 4 can be of a different type from those described above. For example, a spring may be provided to keep the handles away from one another, and hence the respective shells closed. The shells can be then opened by gripping the handles 25 and clenching one's hand. Therefore, a stop means such as a rigid ring with adjustable slot can be employed to maintain the handles in position against the thrust force imparted by the spring.

The inserting means 3 can be without the stopping flange 34 if the longitudinal rib 35 is designed such as to act also as a stopping means besides centring means. Furthermore, said means can have the connecting portion 33 as a hollow cylinder, i.e. without the groove 36.

Furthermore, the device 1 of the invention can comprise housing means (not shown in the figures) suitable to receive and releasably house lighting means to facilitate the visibility of the organ to the operator. Housing and lighting means can be such as those described in the Italian patent No. 1234169 of the same applicant and incorporated herein by reference.

Particularly, the housing means can be represented by a seat formed in the thickness of handle 25 of second shell 22 and such as to house a light source of the pen-type for example. The pen source can be releasably secured to the handle 25 for example by snap engagement such as described in said patent.

Preferably, the handle 25 can be internally provided with light-guiding means such as those described, again, in said patent optionally built in or coated with a dark material such as not to blind the operator and at the same time to focus the light along the channel created by the first 21 and second 22 shells.

## Claims

1. Medical device (1) for examinations /operations of organs comprising dilating means (2) suitable to be inserted in an organ and maintain it wide open and inserting means (3) suitable to facilitate the insertion of said device in said organ, wherein said dilating means comprise a first (21) and a second (22) hollow shells connected such as to move relative to one another and wherein said inserting means comprise a releasably inserting core in said dilating means and comprising an end portion (31) having a rounded shape to facilitate the insertion of said dilating means in said organ.

2. Medical device (1) according to claim 1, wherein said first (21) and second (22) shells each comprise a first free end (23) which, when the shells are in the closed position, remains open.

3. Medical device (1) according to claim 1 or 2, wherein said first (21) and second (22) shells have a substantially cylindrical shape.

4. Medical device (1) according to any claims 2 and 3, wherein said first (21) and second (22) shells comprise a notch (27) formed in said free end (23).

5. Medical device (1) according to any claim 1 to 4, wherein said first (21) and second (22) shells further comprise a second end (24) integrally connected with a handle (25) by means of a joining portion (26).

6. Medical device (1) according to any claim 1 to 5, wherein said first (21) and second (22) shells are connected by means of a pin-slot engagement.

7. Medical device (1) according to claim 6, wherein said pin-slot engagement is provided with two pins (29), carried by respective wings (28) which are formed in the joining portion (26) of said second shell (22), and two slots (29') formed in the joining portion (26) of said first shell (21).

8. Medical device (1) according to any claim 5 to 7, wherein said joining portion (26) of said first shell (21) comprises a through hole (26') to insert said inserting means (3).

9. Medical device (1) according to claim 8, wherein the edge of said through hole (26') comprises a notch (30).

10. Medical device (1) according to any claim 5 to 9, wherein said handle (25) of said first shell (21) is provided by a through hole (25') provided with a tooth (25'').

11. Medical device (1) according to any claim 1 to 10, wherein said inserting means (3) comprise a first end (31) having rounded shape, a second end (32) and a connecting portion (33) between said first and second ends.

12. Medical device (1) according to claim 11, wherein said second end (32) comprises a flange (34).

13. Medical device (1) according to claim 11 or 12, wherein said second end (32) comprises a rib (35) cooperating with the groove 30 of the joining portion (26) of first shell (21) to provide centring means of the inserting means (3) in the dilating means (2).

14. Medical device (1) according to any claim 11 to 13, wherein said connecting portion (33) is provided with a groove (36).

15. Medical device (1) according to any claim 1 to 14, further comprising locking means (4) to lock said first (21) and second (22) shells in the open position.

16. Medical device (1) according to claim 15, wherein said locking means (3) comprise a ring (41) integrally connected with a rigid pin (42) and a nut (44) of the wing-type.

17. Medical device (1) according to claim 16, wherein said pin (42) comprises a longitudinal axial base (46) from which the circle-wedge-shaped teeth (47) protrude, which define a series of grooves (43).

18. Medical device (1) according to claim 17, wherein said base (46) comprises in a substantially diametrically opposite position to said teeth (47) a shoulder (48) suitable to act as the stopping means for said nut (44).

19. Medical device (1) according to any claim 16 to 18, wherein said nut (44) is provided with a radially extending relief (45) and is suitable to be engaged with one of said grooves (43) of said pin (42).

20. Medical device (1) either disposable or multi-use according to any preceding claim **characterized by** being formed with plastic materials such as polypropylene, polystyrene, HD polyethylene or metallic material such as stainless steel, metal alloys for chirurgical instruments.

21. Medical device (1) according to any claim 1 to 20, further comprising housing means for lighting means.

22. Medical device (1) according to claim 21, wherein said housing means comprise a seat formed in said first (21) or second (22) shells.

23. Medical device (1) according to claim 21 or 22, wherein said lighting means are pen-lighting means.

24. Medical device (1) according to any claim 21 to 23, further comprising light-guiding means.
